# EUROPEAN PATENT APPLICATION

(11) **EP 4 717 268 A1**
(43) Date of publication of application: **01.04.2026**
(21) Application number: 24203702.6
(22) Date of filing: 30.09.2024
(51) Int. Cl.: A61K 31/795, A61P 1/00

(54) **PARTICULARLY APPROPRIATE THIOL-FUNCTIONALIZED POLYGLYCEROL DERIVATIVES AND THEIR MEDICAL USES**

(71) Applicant: Freie Universität Berlin, 14195 Berlin (DE)
(72) Inventor: ARENHOEVEL, Justin., 12157 Berlin (DE); HAAG, Rainer., 12209 Berlin (DE)
(74) Representative: Maikowski & Ninnemann Patentanwälte Partnerschaft mbB

(57) **Abstract**

The invention relates to specific polyglycerol derivatives and their further medical uses, namely to dPG/IPG-DTBA and dPG/IPG-cysteamine. These polyglycerol derivatives have a linear or dendritic polyglycerol backbone and carry at least one thiol group covalently bound to the polyglycerol backbone. The claimed medical uses are the use as mucolytic; the use in treating chronic sinusitis, asthma, chronic bronchitis, cystic fibrosis, chronic obstructive pulmonary disease, emphysema, or bronchiectasis; and the use in treating chronic inflammatory bowel diseases, constipation, gastrointestinal malabsorption syndrome, irritable bowel syndrome, steatorrhea or diarrhea.

## Description

The present invention relates to medical uses of particularly appropriate polyglycerol derivatives according to claims 1, 2 and 3, to these thiol-functionalized polyglycerol derivatives themselves according to claim 6, and to a method for manufacturing such thiol-functionalized polyglycerol derivatives according to claim 8.

The human mucosal surfaces (mucosa) are covered with a thin hydrobiopolymer called mucus that has important homeostatic functions and protects those surfaces from dehydration or infection. Mucus consists of highly glycosylated mucin monomers (up to 80 % sugar content), which allows hydrogels to form in the presence of water. These mucin monomers in turn can form disulfide bridges by oxidation and thus multimerize.

Under physiological conditions, mucus solid matter concentrations of approximately 10 mg/ml (percentage of dry mass with respect to moist mass) are found in humans. This concentration can increase massively (up to 80 to 100 mg/ml) in some diseases such as cystic fibrosis (CF), chronic obstructive pulmonary disease (COPD) or bronchial asthma. The high mucus concentration and the formation of disulfide bridges caused by oxidation processes lead to a massive increase in viscoelastic material properties. Affected patients suffer from up to life-threatening shortness of breath, frequent infections and inflammation of the respiratory tract.

For the treatment of such obstructive pulmonary diseases, research is being carried out not only on disease-specific drugs but also on symptomatically effective mucolytic substances. The basic strategy for relieving mucus-induced shortness of breath is to decompose the mucus by reducing the disulfide bridges. N-acetylcysteine (NAC) is the only clinically approved drug with such a mode of action to date, but the effectiveness of the drug has been doubted in several independent studies (Millar, Ann B., et al. "Effect of oral N-acetylcysteine on mucus clearance." British Journal of Diseases of the Chest79 (1985): 262-266.). Patients treated with hypertonic saline solution experienced similar symptom relief as patients treated with NAC.

WO 2021/058818 A1 discusses in detail prior art documents relating to mucolytics of different chemical structures and prior art documents relating to polyglycerol derivatives. In addition, WO 2021/058818 A1 discloses polyglycerol derivatives having a linear or dendritic polyglycerol backbone and carrying at least one thiol group covalently bound to the polyglycerol backbone as well as various medical uses of such polyglycerol derivatives.

It is an object of the present invention to provide polyglycerol derivatives generally complying with the chemical definition as set forth in WO 2021/058818 A1 but being particularly appropriate to be used in novel medicaments that are appropriate for chemically modifying and thus destabilizing mucus, i.e. that act as mucolytic.

This object is achieved by the further medical use, in particular the therapeutic use, of a polyglycerol derivative as mucolytic. Thereby, this polyglycerol derivative comprises a linear or dendritic polyglycerol backbone and carries at least one thiol group covalently bound to the polyglycerol backbone. The thiol group can be bound directly or indirectly to the polyglycerol backbone. In this context, the polyglycerol derivative corresponds to the following general formula (I): 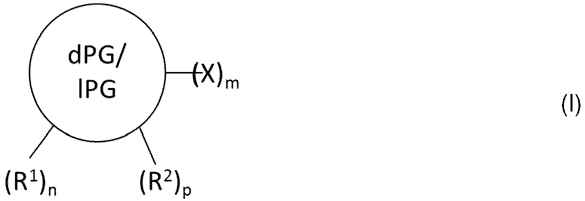 wherein
- dPG: denotes a dendritic polyglycerol backbone,
- IPG: denotes a linear polyglycerol backbone,
- X: is a residue chosen from the group consisting of:

| Structure | Name of polyglycerol compound |
|---|---|
| 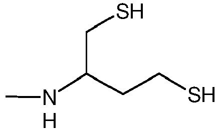 | dPG(S)-DTBA |
| | IPG(S)-DTBA |
| 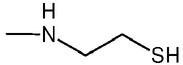 | dPG(S)-cysteamine |
| | IPG(S)-cysteamine |

- R¹, R²: are independently from each other -OH or -OSO₃⁻Z⁺,
- Z: is a single-charge cationic counter ion,
- m: is 1, 2, 3, 4, 5, 6, 7, 8, 9, 10, 11, 12, 13, 14, 15, 16, 17, 18, 19, or 20 for dPG and 1 or 2 for IPG,
- n: is an integer between 5 and 5000, and
- p: is an integer between 0 and 5000.

It is known that thiol-functionalized hydrophilic polyglycerols can effectively penetrate the mucus gel and break disulfide bridges. If the polyglycerols additionally carry sulfate groups, they also have anti-inflammatory properties that are helpful in curing inflammations that might be causative for an excessive mucus production or that might occur in the course of excessive mucus production.

While the DTBA and cysteamine derivatives of dPG and IPG fall under the general definition according to WO 2021/058818 A1 (they represent polyglycerol derivatives comprising a linear or dendritic polyglycerol backbone and carrying at least one thiol group covalently bound to the polyglycerol backbone), they are not explicitly disclosed in that international patent application. Therefore, the present disclosure constitutes a selection invention from the broader disclosure of WO 2021/058818 A1.

Surprisingly, the DTBA and cysteamine derivatives of dPG and IPG show superior and unexpected properties in comparison to other compounds falling under the general definition of WO 2021/058818 A1. E.g., they have a high mucolytic effect, but do not completely destroy the mucus. In addition, they have an unexpected long-term efficacy and a surprisingly low cytotoxicity so that they can be used in higher concentrations than other comparable compounds. This will be further explained below with respect to exemplary embodiments and accompanying Figures.

In an aspect, the invention relates to the further medical use of the same polyglycerol derivative as outlined above for use in reducing mucus viscosity.

In an aspect, the invention relates to the further medical use of the same polyglycerol derivative as outlined above for use in treating mucinous neoplasias, e.g., pseudomyxoma peritonei or mucinous adenocarcinoma.

In an embodiment of the different novel medical uses, the polyglycerol backbone carries a plurality of sulfate groups, wherein a degree of sulfation of the polyglycerol backbone is between 10 and 100 %. Particularly appropriate degrees of sulfation are between 50 % and 100 %, between 70 % and 100 %, between 85 % and 100 %, and in particular between 90 % and 100 %. By sulfating the polyglycerol derivatives, anti-inflammatory properties are transferred onto the polyglycerol derivatives. Thereby, this effect is particularly pronounced if the degree of sulfation is at least 70 %. The degree of sulfation indicates the relative amount of hydroxyl groups of the polyglycerol derivative that have been exchanged by sulfate groups.

In an embodiment of the different novel medical uses, the polyglycerol backbone is biodegradable. This can be achieved, e.g., by manufacturing the polyglycerol backbone by a reaction randomly combining glycerol units and ε-caprolactone units. Such a manufacturing method is disclosed, e.g., in WO 2019/096782 A1. Other biodegradable architectures of the polyglycerol backbone of the polyglycerol derivatives can also be realized.

In an embodiment of the different novel medical uses, the at least one thiol group is directly bound to the polyglycerol backbone without any intermediate linker. In another embodiment of the different novel medical uses, the at least one thiol group is indirectly bound to the polyglycerol backbone via a linker. In this regard, different linkers are generally possible.

In an embodiment, the cationic counter ion is an ion of an alkali metal, in particular a sodium ion (Na⁺) or a potassium ion (K⁺).

In an embodiment, n is an integer between 10 and 4000, in particular between 15 and 3500, in particular between 20 and 3000, in particular between 50 and 2500, in particular between 100 and 2000, in particular between 200 and 1500, in particular between 500 and 1000.

In an embodiment, p is an integer between 10 and 4000, in particular between 15 and 3500, in particular between 20 and 3000, in particular between 50 and 2500, in particular between 100 and 2000, in particular between 200 and 1500, in particular between 500 and 1000.

In an embodiment, the polyglycerol backbone is a dendritic polyglycerol backbone (dPG).

In an aspect, the present invention does not only relate to novel medical uses of polyglycerol derivatives, but to these polyglycerol derivatives themselves. In this aspect, the invention relates to a polyglycerol derivative corresponding to the following general formula (I): 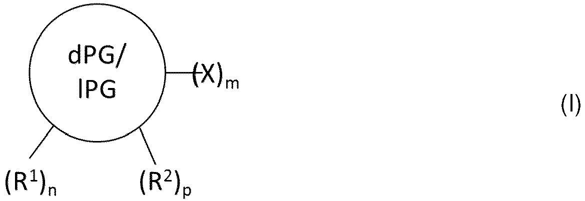 wherein
- dPG: denotes a dendritic polyglycerol backbone,
- IPG: denotes a linear polyglycerol backbone,
- X: is a residue chosen from the group consisting of:

| Structure | Name of polyglycerol compound |
|---|---|
| 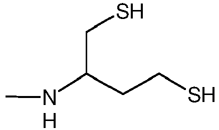 | dPG(S)-DTBA |
| | IPG(S)-DTBA |
| 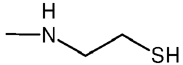 | dPG(S)-cysteamine |
| | IPG(S)-cysteamine |

- R¹, R²: are independently from each other -OH or -OSO₃⁻Z⁺,
- Z: is a single-charge cationic counter ion,
- m: is 1, 2, 3, 4, 5, 6, 7, 8, 9, 10, 11, 12, 13, 14, 15, 16, 17, 18, 19, or 20 for dPG and 1 or 2 for IPG,
- n: is an integer between 5 and 5000, and
- p: is an integer between 0 and 5000.

In an embodiment, the cationic counter ion is an ion of an alkali metal, in particular a sodium ion (Na⁺) or a potassium ion (K⁺).

In an embodiment, the polyglycerol derivative comprises a linear polyglycerol backbone. In an embodiment, the whole polyglycerol backbone of the polyglycerol derivative is linear. The polyglycerol derivative can be denoted as linear polyglycerol derivative (IPG) in these embodiments. In this context, a 1,2-linkage and/or a 1,3-linkage can be established between the individual glycerol units of the polyglycerol derivative.

In an embodiment, the polyglycerol derivative comprises a dendritic polyglycerol backbone. In an embodiment, the whole polyglycerol backbone of the polyglycerol derivative is dendritic. The dendritic backbone of the polyglycerol derivative can also be denoted as hyperbranched backbone. Thus, in these embodiments, the polyglycerol derivative can be denoted as dendritic polyglycerol (dPG) or hyperbranched polyglycerol (hPG). In case of sulfate groups as substituents, the polyglycerol can be denoted as dendritic polyglycerol sulfate (dPGS) or hyperbranched polyglycerol sulfate (hPGS). Hyperbranched polyglycerol sulfates express little or no anticoagulant effect.

In an embodiment, the residue X is:

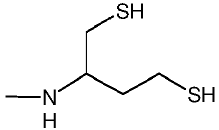

Such IPG-DBTA and in particular such dPG-DBTA compounds have particularly positive effects, as will be explained below in further detail.

In an embodiment, the residue X is:

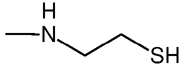

Also such IPG-cysteamine and in particular such dPG-cysteamine compounds have very positive effects, as will be explained below in further detail.

In an aspect, the present invention relates to a method for manufacturing a polyglycerol derivative according to the preceding explanations. This method comprises the steps explained in the following.

In a first step (step a)), an oxidizing agent is added to an aqueous solution of a polyglycerol derivative corresponding to the following general formula (II): 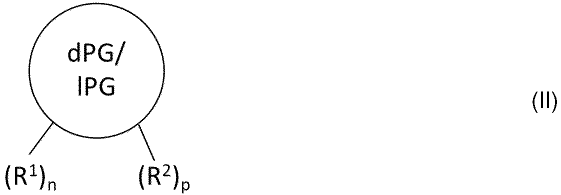 wherein
- dPG: denotes a dendritic polyglycerol backbone,
- IPG: denotes a linear polyglycerol backbone,
- R¹, R²: are independently from each other -OH or -OSO₃⁻Z⁺,
- Z: is a single-charge cationic counter ion,
- n: is an integer between 5 and 5000, and
- p: is an integer between 0 and 5000,

In a second step (step b)), some of the hydroxyl groups of the polyglycerol derivate are oxidized to aldehyde groups, thus obtaining an aldehyde-functionalized polyglycerol derivative,

In a further step (step c)), dithiobutylamine or cysteamine is added to the aldehyde-functionalized polyglycerol derivative. The mixture is allowed to react. In an embodiment, this reaction is allowed to occur during stirring the reaction mixture. An appropriate reaction time lies in a range from 12 hours to 48 hours, in particular from 24 hours to 36 hours.

In a further step (step d)), a reducing agent is added to the product obtained in the previous step (step c)) to obtain a thiol-functionalized polyglycerol derivative corresponding to the following general formula (I): 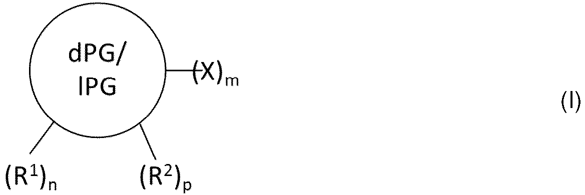 wherein X is:

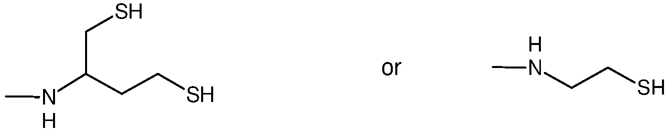

In an embodiment, the polyglycerol derivatives corresponding to general formulae (I) and (II) are dendritic polyglycerol derivatives. This will result in dPG-DTBA or dPG-cysteamine, or in dPGS-DTBA or dPGS-cysteamine if the dPG is sulfated. Thus, the general abbreviation of the resulting product is dPG(S)-DTBA or dPG(S)-cysteamine.

Generally, any common oxidizing agent can be used for introducing aldehyde functionalities into the polyglycerol derivative of general formula (II). Perbromate, perchlorate, and periodate are appropriate oxidizing agents. In an embodiment, the oxidizing agent is a periodate. Sodium periodate is a particularly appropriate oxidizing agent.

Generally, any common reducing agent can be used for removing unreacted aldehyde functionalities from the thiol-functionalized polyglycerol derivative. In an embodiment, the reducing agent is a cyanoborohydride. Sodium cyanoborohydride is a particularly appropriate reducing agent.

In an embodiment, the aldehyde-functionalized polyglycerol derivative has a degree of functionalization lying in a range from 2 % to 20 %, in particular from 3 % to 15 %, in particular from 4 % to 12 %, in particular from 5 % to 10 %. Expressed in other words, 2 % to 20 % of all available hydroxyl groups in the polyglycerol backbone have been oxidized to aldehyde groups or functionalities in this embodiment.

In an embodiment, the dithiobutylamine or the cysteamine is added in a molar ratio of 0.5 to 5, in particular of 1 to 4, in particular of 2 to 3, to the aldehyde functionalities of the aldehyde-functionalized polyglycerol derivative. Expressed in other words, 0.5 to 5 equivalents of dithiobutylamine or cysteamine are added per aldehyde functionality.

In an embodiment, the reducing agent is added in a molar ratio of 5 to 15, in particular of 6 to 14, in particular of 7 to 13, in particular of 8 to 12, in particular of 9 to 11, in particular of 10 to 10.5 to the aldehyde functionalities of the aldehyde-functionalized polyglycerol derivative. Expressed in other words, 5 to 15 equivalents of reducing agent are added per aldehyde functionality.

In an embodiment, a pH value of a solution containing the aldehyde-functionalized polyglycerol derivative is adjusted to a value lying in a range from 3 to 6, in particular from 4 to 5, prior to adding the dithiobutylamine or the cysteamine.

In an embodiment, the aldehyde-functionalized polyglycerol derivative is purged with an inert gas such as nitrogen prior to adding the dithiobutylamine or the cysteamine.

In an embodiment, step c) is carried out under stirring for a time period lying in a range from 4 hours to 24 hours, in particular from 6 hours to 18 hours, in particular from 8 hours to 12 hours.

In an embodiment, the crude product obtained in step d) (i.e., the polyglycerol derivative according to general formula (I)), is purified by dialysis, in particular by dialysis against hydrochloric acid such as hydrochloric acid having a concentration lying in a range from 0.1 mM to 10 mM, in particular 0.5 mM to 5 mM, in particular 0.8 mM to 3 mM, in particular 1 mM to 2 mM.

In an aspect, the invention relates to a method of treatment of a human or animal patient in need thereof to achieve a reduction of the viscosity of mucus of the patient. Thus, this method aims in reducing the amount of mucus in or on an organ of the patient by enabling an easier decomposition and discharge of the mucus. This method is carried out by administering a polyglycerol derivative having a linear or dendritic polyglycerol backbone and carrying at least one thiol group covalently bound to the polyglycerol backbone to the patient. In this context, the polyglycerol derivative corresponds to the following general formula (I): 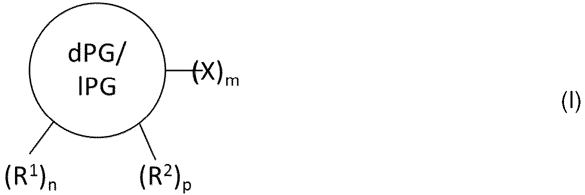 wherein
- dPG: denotes a dendritic polyglycerol backbone,
- IPG: denotes a linear polyglycerol backbone,
- X: is a residue chosen from the group consisting of:

| Structure | Name of polyglycerol compound |
|---|---|
| 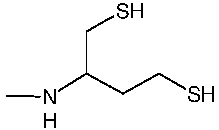 | dPG(S)-DTBA |
| | IPG(S)-DTBA |
| 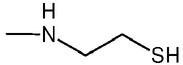 | dPG(S)-cysteamine |
| | IPG(S)-cysteamine |

- R¹, R²: are independently from each other -OH or -OSO₃⁻Z⁺,
- Z: is a single-charge cationic counter ion,
- m: is 1, 2, 3, 4, 5, 6, 7, 8, 9, 10, 11, 12, 13, 14, 15, 16, 17, 18, 19, or 20 for dPG and 1 or 2 for IPG,
- n: is an integer between 5 and 5000, and
- p: is an integer between 0 and 5000.

In an aspect, the invention relates to a method of treatment of a human or animal patient suffering from chronic sinusitis, asthma, chronic bronchitis, cystic fibrosis, chronic obstructive pulmonary disease, emphysema, or bronchiectasis and being in need of such treatment by administering the same polyglycerol derivative as defined in the preceding above to the patient.

In an aspect, the invention relates to a method of treatment of a human or animal patient suffering from chronic inflammatory bowel diseases, constipation, gastrointestinal malabsorption syndrome, irritable bowel syndrome, steatorrhea or diarrhea and being in need of such treatment by administering the same polyglycerol derivative as defined in two paragraphs above to the patient.

All embodiments explained with respect to the different novel medical uses of the polyglycerol derivatives can be combined in any desired manner and can be transferred to the respective other uses. Furthermore, these embodiments can be transferred in any desired combination to the described polyglycerol derivatives themselves and to the described methods. Likewise, the embodiments of the polyglycerol derivatives can be combined in any desired manner and can be transferred to the described uses and to the described methods. Additionally, the embodiments of the described methods can be combined in any desired manner and can be transferred to the other methods, to the described uses and to the polyglycerol derivatives.

Further details of aspects of the present invention will be explained with respect to exemplary embodiments and accompanying Figures. In the Figures:
- Figure 1A: illustrates the relative oxidation stability of dPG-DTBA;
- Figure 1B: illustrates the number of cleaved disulfides per entity of reducing agent;
- Figure 2A: illustrates the cytotoxicity of dPG-DTBA;
- Figure 2B: illustrates the cytotoxicity of DTBA;
- Figure 2C: illustrates the cytotoxicity of dPGS-SH;
- Figure 2D: illustrates the cytotoxicity of DTBA, dPG-DTBA, and dPGS-SH;
- Figures 3A and 3B: illustrate the ability of dPG-DTBA to reduce mucus-inspired disulfide hydrogel; and
- Figures 4A and 4B: illustrate the effect of N-acetylcysteine, dPG-cysteamine and dPG-DTBA on the high molecular weight compounds of airway mucins.

dPG-DTBA and dPG-cysteamine were manufactured according to the following procedure: Polyglycerols or polyglycerol sulfates were oxidized with sodium periodate in aqueous solution to obtain aldehyde functionalities in a degree of functionalization (DF) of 2 % to 20 %. Aldehyde functionalized polymers were dissolved in H₂O (10 mg/ml), the pH was adjusted to 5, and the solution was carefully purged with nitrogen. Dithiobutylamine (DTBA) or cysteamine (2 eq. per aldehyde functionality) were added and the mixture was stirred for 24 hours under oxygen exclusion. Sodium cyanoborohydride (10 eq. per aldehyde functionality) was added and the reaction mixture was stirred for additional 8 hours. The crude product was dialyzed in 1 mM HCl; dPG-DTBA or dPG-Cysteamine were obtained as colorless salts.

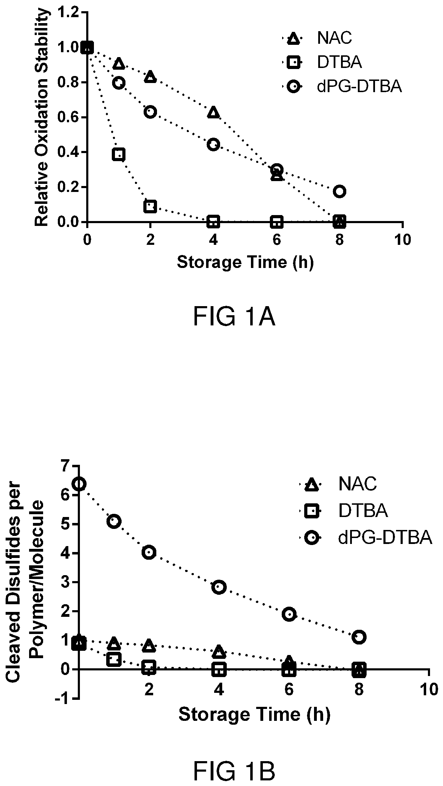

Figures 1A and 1B illustrate the relative oxidation stability of dPG-DTBA in comparison to DTBA and N-acetylcysteine (NAC). dPG-DTBA, DTBA and NAC were stored at a concentration of 0.1 mM in 10 mM phosphate-buffered saline (PBS) buffer at pH 7.4 and at 37 °C for up to 8 hours. The remaining thiol groups after storage were quantified using the Ellman's assay by detecting the absorbance of cleaved DTNB units (TNB²⁻, 412 nm). Quantification was achieved by a standard calibration using NAC as a reference. Figure 1A illustrates that the number of remaining thiol groups in the dPG-DTBA after 8 hours storage is significantly higher than the number of remaining thiol groups in NAC or DTBA, respectively. Thus, dPG-DTBA is less prone to autoxidation than DTBA or NAC; it remains its ability to reduce disulfides after having been stored in PBS at 37° (i.e., at body temperature). Expressed in other words, dPG-DTBA remains longer active within a patient's body; it has an extended time of action.

Figure 1B shows the number of cleaved disulfides per entity of reducing agent. 5,5'-dithiobis-(2-nitrobenzoic acid) (Ellman's reagent; DTNB) was used as a model disulfide. The number of cleaved disulfides was determined by measuring the absorbance at 412 nm in reference to a standard calibration of a molecule with known number of thiol groups. It can be seen from Figure 1B that each dPG-DTBA molecule can reduce more than six disulfides and has a reductive capacity after 8 hours of storage in PBS buffer at 37 °C that is even higher than the reductive capacity of NAC or DTBA without any storage. Thus dPG-DTBA is a highly potent reducing agent exhibiting a long-term efficacy.

The disulfide reduction mechanism of dPG-DTBA differs from monothiol motives (e.g., NAC or dPGS-SH) since the reduction is thermodynamically favored by an intramolecular ring formation of the reducing agent. This results in a strong disulfide redox potential (-302 ± 0.6) mV, as determined by HPLC analysis of the equilibrated reaction with oxidized DTT (data not shown). Moreover, this difference in reduction mechanism excludes the unwanted side-feature of monothiol motives acting as a mucin crosslinker, which cannot be neglected for monothiol-functionalized polymers having more than one functional group (e.g., dPGS-SH).

The physico-chemical properties of dPG-DTBA and dPG-cysteamine are summarized in the following table and compared with the respective properties of dPG-cysteine that is known from WO 2021/058818 A1:

**Table 1. Physico-chemical properties of different thiolated polyglycerol variants.**

| **Polymer** | **Molecular weight [kDa]¹** | **Hydrodynamic diameter [nm]²** | **Degree** of **functionalization [%]¹** | **Reduced disulfides per polymer³** |
|---|---|---|---|---|
| dPG-cysteine | 11.3 | 5.0 ± 0.2 | 3.0 | 4.3 ± 0.1 |
| dPG-cysteamine | 11.5 | 4.3 ± 0.3 | 4.1 | 5.8 ± 0.1 |
| dPG-DTBA | 13.0 | 4.6 ± 0.4 | 6.3 | 6.2 ± 0.1 |

| | | | | |
|---|---|---|---|---|
| ¹ determined by ¹H NMR ²determined by dynamic light scattering in 10 mM PBS (pH = 7.4) ³ determined by using the model disulfide DTNB | | | | |

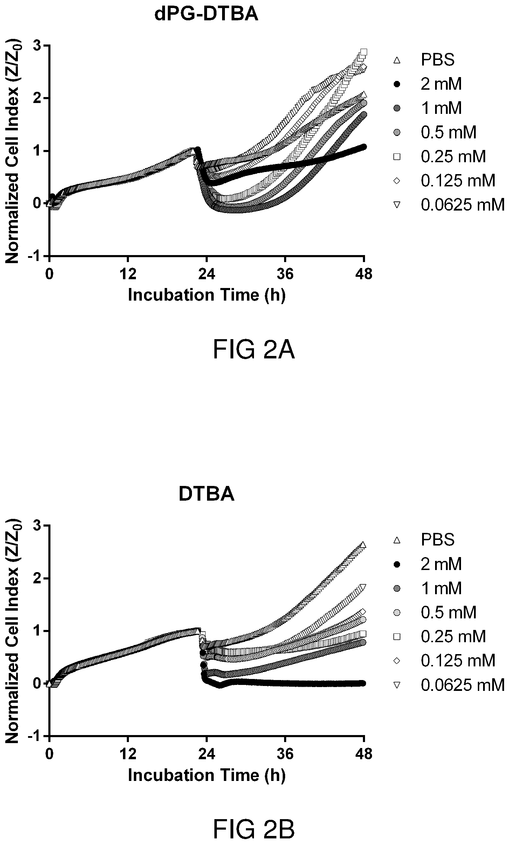

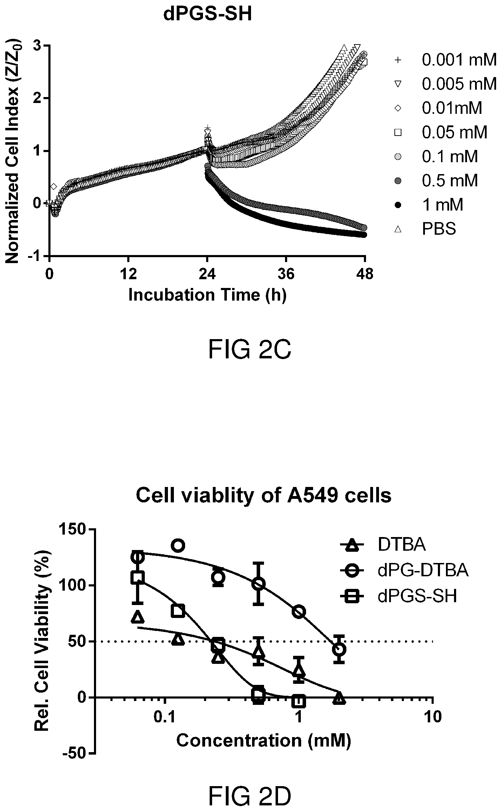

Figures 2A to 2C illustrate the cell viability of A549 cells in the presence of dPG-DTBA or DTBA. Figures 2A and 2B show representative real-time cell analysis (RTCA) plots of A549 cells treated with dPG-DTBA (Figure 2A), with DTBA (Figure 2B) or with dPGS-SH (Figure 2C) in different concentrations (0.0625 mM to 2 mM). PBS was used as negative control. The cells were incubated until a cell index of 1 was reached prior to the addition of dPG-DTBA or DTBA.

Figure 2D compares the cytotoxicity of DTBA, dPG-DTBA, and dPGS-SH in relation to PBS-treated A549 cells. The data was acquired from the RTCA plots shown in Figures 2A to 2C. The cell viability was determined from the measured cell index 24 hours after treatment (n = 4). The cell viability was additionally evaluated by microscope images of A549 cells taken 48 hours after treatment with 2 mM, 1 mM, or 0.5 mM DTBA, dPG-DTBA, or dPGS-SH (not shown).

The results shown in Figures 2A to 2D illustrate that dPG-DTBA has a significantly higher biocompatibility than DTBA or dPGS-SH; dPG-DTBA showed a cytotoxicity concentration (CC50) of 1.8 mM, whereas DTBA showed a CC50 of 0.2 mM. Even after treatment of cells with 2 mM dPG-DTBA, more than 40 % cell viability could be observed. In contrast, DTBA did not reach 100 % cell viability even at the lowest tested concentration (0.0625 mM). It was completely surprising that a cytotoxic molecule loses its cytotoxicity upon conjugation with a polyglycerol backbone or core such as dPG or IPG.

dPGS-SH (as disclosed in WO 2021/058818 A1) showed under the same experimental setup a CC50 value of 0.2 mM, approximately similar to that of DTBA. However, the cell viability of cells treated with dPG-DTBA was more than 9 times higher than that of cells treated with dPGS-SH. This low cytotoxicity of dPG-DTBA was very surprising. At the same time, it enables a huge range of applications of dPG-DTBA in various concentrations and thus makes this compound to a particularly appropriate candidate for the medical uses mentioned above. In a preliminary study, IPG-DTBA showed a similar low cytotoxicity like dPG-DTBA so that the findings presented herein also relate to IPG-DTBA.

It should be noted that a treatment of cells with 1 mM dPG-DTBA corresponds to an applied dithiol concentration of 6.2 mM (confer Figure 1B for more details), whereas the applied dithiol concentration of DTBA corresponds 1:1 to the factually applied dithiol concentration. Similarly, the applied thiol concentration of dPGS-SH corresponds 4:1 to the factually applied dithiol concentration. Thus, the higher efficacy of dPG-DTBA even increase the positive effect on the cell viability of this compound since it needs to be used only in a lower concentration to achieve the same reducing effect as other substances such as DTBA or dPGS-SH. Expressed in other words, the higher efficacy and the lower cytotoxicity of dPG-DTBA synergistically act together when using this compound for medical applications.

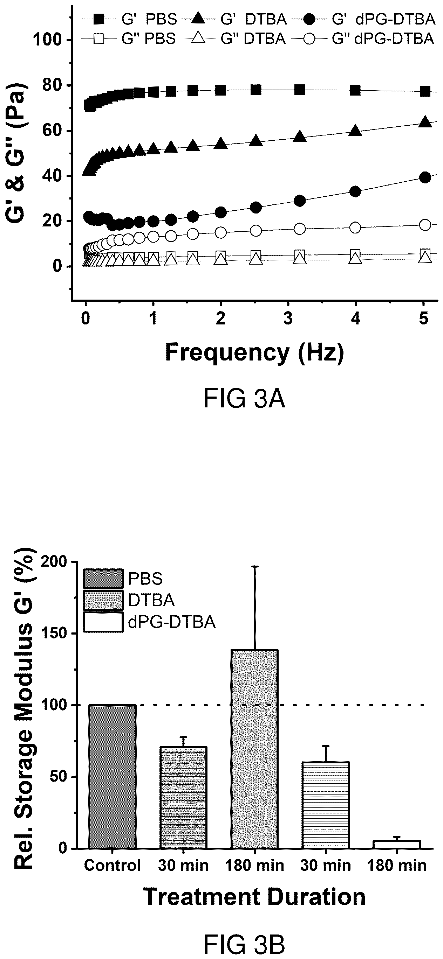

Figures 3A and 3B illustrate the ability of dPG-DTBA to reduce mucus-inspired disulfide hydrogel (MIH) (prepared according to the method disclosed by Bej et al. (Bej, Raju, et al. "Mucus-Inspired Self-Healing Hydrogels: A Protective Barrier for Cells against Viral Infection." Advanced Materials (2024): 2401745)).

Figure 3A shows representative frequency sweeps on MIH after 30 min treatment with PBS (negative control), 1 mM dPG-DTBA, or 1 mM DTBA. Figure 3B shows the relative storage modulus of MIH after treatment with PBS (negative control; first bar), 1 mM DTBA (second and third bar), or 1 mM dPG-DTBA (fourth and fifth bar) after 30 minutes and after 180 min. It can well be seen that dPG-DTBA is able to reduce the disulfide bridges in MIH better than DTBA already after 30 minutes. After 180 minutes of incubation, the difference is even much stronger. While the reductive capacity of DTBA is not present anymore, the storage modulus of dPG-DTBA-treated MIH is significantly reduced, indicating an almost complete destruction of the disulfide network previously present in MIH. These findings were confirmed by visual inspection of the treated hydrogels (data not shown). The DTBA-treated sample showed a solid hydrogel similar to the PBS control. In contrast, the dPG-DTBA-treated hydrogel showed a liquid like behavior indicating the reduction of the previously present disulfide bonds.

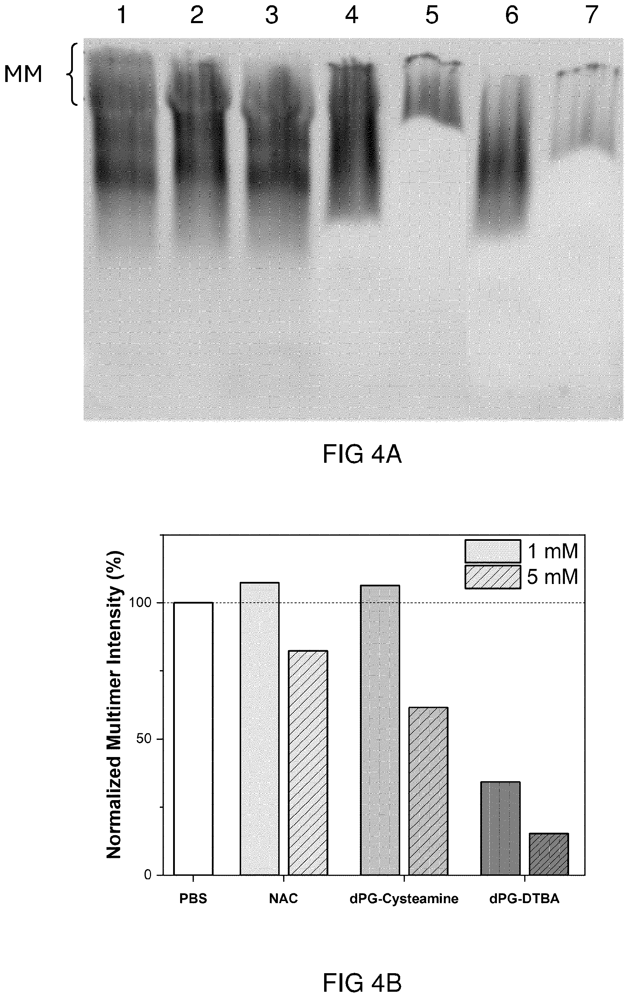

Figures 4A and 4B illustrate the effect of NAC, dPG-cysteamine, and dPG-DTBA on the high molecular weight compounds of airway mucins. Figure 4A shows Western blot images of MUC5B, whereas Figure 4B illustrates a quantification of multimer intensity of MUC5B Western blots normalized to PBS-treated controls. In the Western blot of Figure 4A, the following samples were analyzed:

| | |
|---|---|
| lane 1: | PBS (negative control) |
| lane 2: | 1 mM NAC |
| lane 1: | 5 mM NAC |
| lane 1: | 1 mM dPG-cysteamine |
| lane 1: | 5 mM dPG-cysteamine |
| lane 1: | 1 mM dPG-DTBA |
| lane 1: | 5 mM dPG-DTBA |

"MM" in Figure 4A denotes "mucin multimers". These mucin multimers can also be denoted as MUC5B.

As illusrated in Figure 4B, dPG-cysteamine showed a clear reduction of the mucin multimer network at a concentration of 5 mM, whereas dPG-DTBA showed an even stronger effect already at a low concentration of 1 mM. At a concentration 5 mM, dPG-DTBA reduced the mucin multimers to an even higher extent.

Summarized, after 30 minutes of treatment of human sputum samples from CF patients with dPG-DTBA and dPG-cysteamine, a reduction of the MUC5B multimer state due to reduction of disulfide crosslinks was achieved. dPG-DTBA is - in contrast to dPGS-SH - already effective at a concentration that is non-toxic for cells in real-time cell analysis of A549 cells. Moreover, the treatment of CF sputa with 1 mM dPGS-SH showed a reduction of MUC5B multimer intensity of approximately 25 % compared to PBS treated control as published in Arenhoevel et. al. (Arenhoevel, Justin et al. "Thiolated polyglycerol sulfate as potential mucolytic for muco-obstructive lung diseases" Biomaterials Science 12 (2024): 4376-4385). The treatment of dPG-DTBA led to a reduction of approximately 60% in the same experimental setup. But also dPG-cysteamine showed a very potent reductive efficacy at a concentration of only 5 mM and was thus significantly better in reducing mucin multimers than the current "gold standard" NAC. It should be noted that dPG-Cysteamine and dPG-DTBA are capable to reduce multiple disulfides and therefore have the potential for a stronger mucolytic effect and a longer time of action.

### List of references cited in the preceding sections or considered otherwise to be relevant

1. Bej, Raju, et al. "Mucus-Inspired Self-Healing Hydrogels: A Protective Barrier for Cells against Viral Infection." Advanced Materials (2024): 2401745
2. WO 2021/058818 A1
3. US 2005/0260140 A1
4. US 7,345,051 B2
5. US 2016/023149 A1
6. DE 26 02 196 A1
7. US 2015/0307530 A1
8. WO 98/38995 A1
9. US 4,210,666 A
10. US 3,567,835 A
11. US 9,346,753 B2
12. WO 2016/123335 A1
13. WO 2016/176423 A1
14. Lee, Rees L., et al. "Thioredoxin and dihydrolipoic acid inhibit elastase activity in cystic fibrosis sputum." American Journal of Physiology-Lung Cellular and Molecular Physiology 289.5 (2005): L875-L882
15. Yuan, Shaopeng, et al. "Oxidation increases mucin polymer cross-links to stiffen airway mucus gels." *Science translational medicine* 7.276 (2015): 276ra27-276ra27
16. Tabachnik, Nina Felice, et al. "Protein binding of N-2-mercaptoethyl-1, 3-diaminopropane via mixed disulfide formation after oral administration of WR 2721." *Journal of Pharmacology and Experimental Therapeutics* 220.2 (1982): 243-246
17. Calderón, Marcelo, et al. "Dendritic polyglycerols for biomedical applications." Advanced Materials 22.2 (2010): 190-218
18. Gröger, Dominic, et al. "Synthesis and biological evaluation of radio and dye labeled amino functionalized dendritic polyglycerol sulfates as multivalent anti-inflammatory compounds." Bioconjugate Chemistry 24.9 (2013): 1507-1514
19. Leichner, Christina, et al. "Thiolated polymers: Bioinspired polymers utilizing one of the most important bridging structures in nature." Advanced Drug Delivery Reviews 151 (2019): 191-221
20. Leitner, Verena M., et al. "Thiolated polymers: evidence for the formation of disulphide bonds with mucus glycoproteins." European Journal of Pharmaceutics and Biopharmaceutics 56.2 (2003): 207-214
21. EP 0 045 285 A1
22. WO 2014/153009 A2
23. Ehre, Camille, et al. "An improved inhaled mucolytic to treat airway muco-obstructive diseases." American Journal of Respiratory and Critical Care Medicine 199.2 (2019): 171-180
24. Arenhoevel, Justin et al. "Thiolated polyglycerol sulfate as potential mucolytic for muco-obstructive lung diseases" Biomaterials Science 12 (2024): 4376-4385

## Claims

1. Polyglycerol derivative, having a linear or dendritic polyglycerol backbone and carrying at least one thiol group covalently bound to the polyglycerol backbone for medical use as mucolytic,
**characterized**
**in that** the polyglycerol derivative corresponds to the following general formula (I): 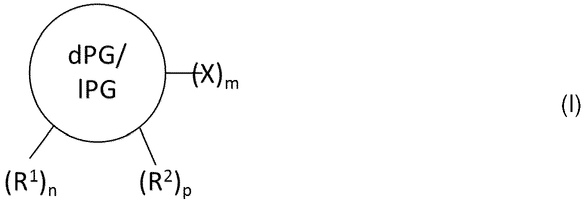
wherein
dPG denotes a dendritic polyglycerol backbone,
IPG denotes a linear polyglycerol backbone,
X is a residue chosen from the group consisting of: 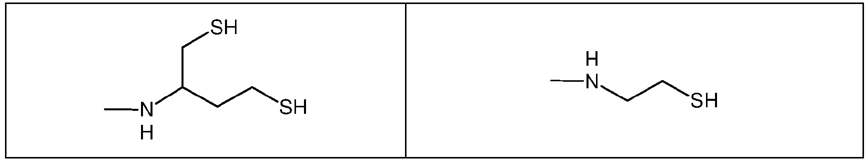
R¹, R² are independently from each other -OH or -OSO₃⁻Z⁺,
Z is a single-charge cationic counter ion,
m is an integer between 1 and 20 for dPG and 1 or 2 for IPG,
n is an integer between 5 and 5000, and
p is an integer between 0 and 5000.

2. Polyglycerol derivative, having a linear or dendritic polyglycerol backbone and carrying at least one thiol group covalently bound to the polyglycerol backbone for use in treating chronic sinusitis, asthma, chronic bronchitis, cystic fibrosis, chronic obstructive pulmonary disease, emphysema, or bronchiectasis,
**characterized**
**in that** the polyglycerol derivative corresponds to the following general formula (I): 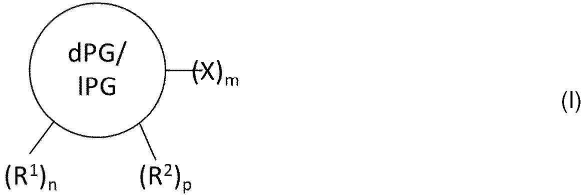
wherein
dPG denotes a dendritic polyglycerol backbone,
IPG denotes a linear polyglycerol backbone,
X is a residue chosen from the group consisting of: 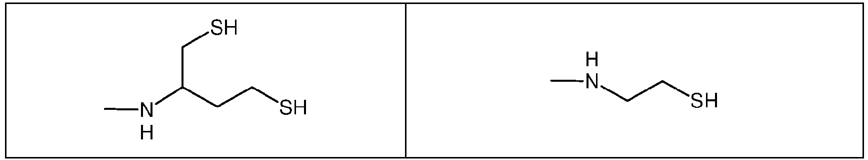
R¹, R² are independently from each other -OH or -OSO₃⁻Z⁺,
Z is a single-charge cationic counter ion,
m is an integer between 1 and 20 for dPG and 1 or 2 for IPG,
n is an integer between 5 and 5000, and
p is an integer between 0 and 5000.

3. Polyglycerol derivative, having a linear or dendritic polyglycerol backbone and carrying at least one thiol group covalently bound to the polyglycerol backbone for use in treating chronic inflammatory bowel diseases, constipation, gastrointestinal malabsorption syndrome, irritable bowel syndrome, steatorrhea or diarrhea,
**characterized**
**in that** the polyglycerol derivative corresponds to the following general formula (I): 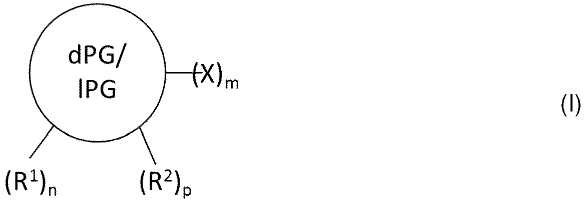
wherein
dPG denotes a dendritic polyglycerol backbone,
IPG denotes a linear polyglycerol backbone,
X is a residue chosen from the group consisting of: 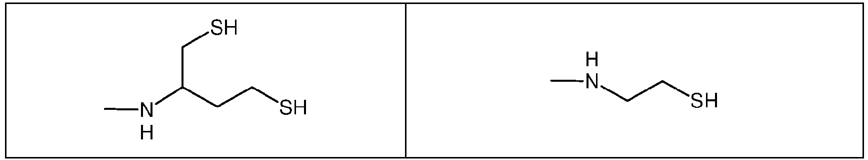
R¹, R² are independently from each other -OH or -OSO₃⁻Z⁺,
Z is a single-charge cationic counter ion,
m is an integer between 1 and 20 for dPG and 1 or 2 for IPG,
n is an integer between 5 and 5000, and
p is an integer between 0 and 5000.

4. Polyglycerol derivative for use according to any of the preceding claims, **characterized in that** the polyglycerol backbone carries a plurality of sulfate groups, wherein a degree of sulfation of the polyglycerol backbone is between 10 and 100 %.

5. Polyglycerol derivative for use according to any of the preceding claims, **characterized in that** the polyglycerol backbone is biodegradable.

6. Polyglycerol derivative corresponding to the following general formula (I): 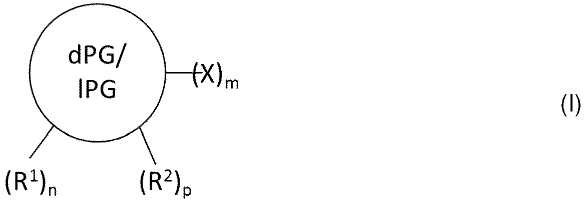 wherein
dPG denotes a dendritic polyglycerol backbone,
IPG denotes a linear polyglycerol backbone,
X is a residue chosen from the group consisting of: 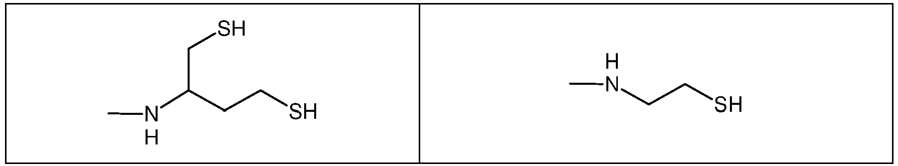
R¹, R² are independently from each other -OH or -OSO₃⁻Z⁺,
Z is a single-charge cationic counter ion,
m is an integer between 1 and 20 for dPG and 1 or 2 for IPG,
n is an integer between 5 and 5000, and
p is an integer between 0 and 5000.

7. Polyglycerol derivative according to claim 6, **characterized in that** the polyglycerol derivative comprises a dendritic polyglycerol backbone.

8. Method for manufacturing a polyglycerol derivative according to claim 6 or 7, **characterized by** the following steps:
a) adding an oxidizing agent to an aqueous solution of a polyglycerol derivative corresponding to the following general formula (II): 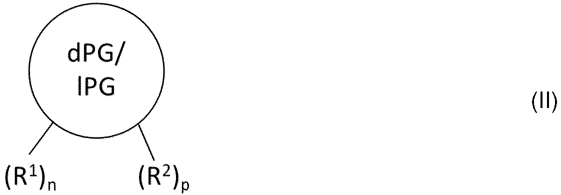 wherein
dPG denotes a dendritic polyglycerol backbone,
IPG denotes a linear polyglycerol backbone,
R¹, R² are independently from each other -OH or -OSO₃⁻Z⁺,
Z is a single-charge cationic counter ion,
n is an integer between 5 and 5000, and
p is an integer between 0 and 5000,
b) oxidizing some of the hydroxyl groups of the polyglycerol derivate to aldehyde groups, thus obtaining an aldehyde-functionalized polyglycerol derivative,
c) adding dithiobutylamine or cysteamine to the aldehyde-functionalized polyglycerol derivative and allowing the mixture to react,
d) adding a reducing agent to the product obtained in step c) to obtain a thiol-functionalized polyglycerol derivative corresponding to the following general formula (I): 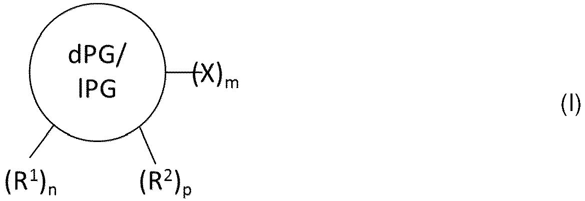 wherein X is:

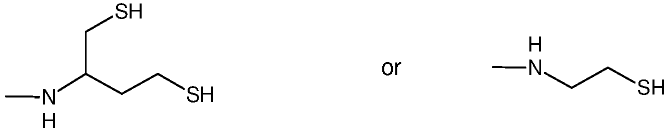

9. Method according to claim 8, **characterized in that** the polyglycerol derivatives corresponding to general formulae (I) and (II) are dendritic polyglycerol derivatives.

10. Method according to claim 8 or 9, **characterized in that** the oxidizing agent is a periodate.

11. Method according to any of claims 8 to 10, **characterized in that** the reducing agent is a cyanoborohydride.

12. Method according to any of claims 8 to 11, **characterized in that** the aldehyde-functionalized polyglycerol derivative has a degree of functionalization lying in a range from 2 % to 20 %.

13. Method according to any of claims 8 to 12, **characterized in that** the dithiobutylamine or the cysteamine is added in a molar ratio of 0.5 to 5 to the aldehyde functionalities of the aldehyde-functionalized polyglycerol derivative.

14. Method according to any of claims 8 to 13, **characterized in that** the reducing agent is added in a molar ratio of 5 to 15 to the aldehyde functionalities of the aldehyde-functionalized polyglycerol derivative.

15. Method according to any of claims 8 to 14, **characterized in that** a pH value of a solution containing the aldehyde-functionalized polyglycerol derivative is adjusted to a value lying in a range from 3 to 6 prior to adding the dithiobutylamine or the cysteamine.
